# EUROPEAN PATENT APPLICATION

(11) **EP 4 144 309 A1**
(43) Date of publication of application: **08.03.2023**
(21) Application number: 21796800.7
(22) Date of filing: 23.04.2021
(51) Int. Cl.: A61B 17/22

(54) **THROMBUS REMOVAL APPARATUS**

(30) Priority: 27.04.2020 CN 202010341446
(71) Applicant: Lifetech Scientific (Shenzhen) Co., Ltd., Shenzhen, Guangdong 518000 (CN)
(72) Inventor: LI, Siyi, Shenzhen, Guangdong 518000 (CN); SHAN, Shuo, Shenzhen, Guangdong 518000 (CN)
(74) Representative: Prinz & Partner mbB
(86) International application number: PCT/CN2021/089168
(87) International publication number: WO 2021/218793

(57) **Abstract**

A thrombus removal apparatus includes a thrombus removal device, (a push-pull member (connected to the thrombus removal device, at least two connecting rods extending from the proximal end to the distal end along the thrombus removal device, and a capture member disposed on the connecting rods.) The thrombus removal device also includes a lumen structure, wherein the connecting rods and the capture member (enclose the lumen structure. The capture member includes a first supporting rod and a second supporting rod, wherein the proximal end of the first supporting rod and the proximal end of the second supporting rod are respectively connected to different connecting rods and form a capture member starting end and a capture member free end. The thrombus removal apparatus solves the problems that the existing thrombus removal apparatuses do not firmly capture thrombi during the process of thrombus removal and have poor wall-attaching performance.

## Description

### Technical Field

The embodiments disclose a medical apparatus and instruments, and relate to a thrombus removal apparatus.

### Background

Acute ischemic stroke (AIS), commonly known as cerebral infarction is a kind of nerve tissue injury caused by ischemic necrosis of regional brain tissues due to sudden obstruction of cerebral blood flow. AIS, the most common type of stroke, is the leading cause of death and disability in the middle aged and elderly people. The acute stroke caused by large vessel occlusion may be an extremely dangerous condition with a high mortality rate and a high disability rate. Strokes may cause great physical and mental harm to patients and also cause heavy burden to patients' family and the society once they occur.

Vascular recanalization is the key to the treatment of acute ischemic stroke. At present, the conventional methods for treating acute ischemic stroke include two major categories: interventional thrombolysis and mechanical thrombectomy. Interventional thrombolysis is that a thrombolytic agent is injected into the nearby of the lesion in the blood vessel by a catheter, so as to instantaneously form a very high concentration of the thrombolytic agent at the lesion locally, accelerating the speed of thrombolysis, thus increasing the chance of vascular recanalization. Thrombolytic therapy is only suitable for small-sized thrombus but has not ideal effect on large-sized thrombus. In acute middle cerebral artery infarction, if the length of thrombus exceeds 8 mm, occluded blood vessel is hardly recanalized by intravenous thrombolysis, and even if the occluded blood vessel can be recanalized, the probability of blood vessel re-occlusion is very high. To solve the above problems, a mechanical device can be used to remove thrombi for the patients beyond the time window of thrombolytic therapy and with contraindications of thrombolytic therapy.

This method can quickly recanalize the occluded blood vessels, improve the rate of vascular recanalization, reduce the dose of thrombolytic drugs, decrease the incidence of symptomatic intracranial hemorrhage, extend the therapeutic time window and shorten the recanalization time, thus fighting for more time for reversible ischemic brain tissues and significantly improving the prognosis of patients.

Currently, the commercially available thrombus removal stents include MERCI^{™}, PENUMBRA^{™}, TREVO^{™}, and SOLITAIRE^{™} among them. These thrombus removal stents serve to embed thrombi into the stents or push thrombi between the blood vessel and the stent by means of radial support force and then take out the thrombi. The way of the thrombus removal stent may have the following consequences: i, when a red thrombus has entered into the stent, the stent's diameter becomes smaller and thus produces a cutting effect on the thrombus, such that the originally larger intact thrombus is cut into multiple smaller-sized thrombi; ii, when the thrombus does not enter into the stent, the stent's diameter becomes smaller, and the squeezing effect of the stent on the thrombus is weakened, and there is friction between the thrombus and the vessel; therefore, the thrombus is easily separated from the stent during the withdrawal process of the stent; iii, the existing thrombus removal device is generally a continuous stent; the deformation of a former portion of the stent will lead to the deformation of the adjacent portions; therefore, the stent cannot maintain good wall-attaching performance when passing through a curved vessel, such that the thrombus is separated from the stent. The above several conditions lead to the weakening of the thrombus removal performance.

### Summary

The embodiments may provide a thrombus removal apparatus; and solve the problems that the existing thrombus removal apparatuses do not firmly capture thrombi during the process of thrombus removal, and have poor wall-attaching performance when passing through a curved blood vessel.

A thrombus removal apparatus, including a thrombus removal device and a push-pull member connected to the thrombus removal device; the thrombus removal device includes a lumen structure, at least two connecting rods extending from the proximal end to the distal end along the thrombus removal device, and a capture member disposed on the connecting rods; the connecting rods and the capture members enclose the lumen structure; where the capture member includes a first supporting rod and a second supporting rod, and the proximal end of the first supporting rod and the proximal end of the second supporting rod are respectively connected to the different connecting rods; and the end where the proximal end of the first supporting rod is located and the end where the proximal end of the second supporting rod is located form a capture member starting end, and the distal end of the first supporting rod and the distal end of the second supporting rod are connected to form a capture member free end.

In one embodiment, at least one of the at least two connecting rods is in a rectilinear shape, a waveform shape or a fold-line shape.

In one embodiment, the capture member has an included angle with an axial cross-section of the thrombus removal device where the capture member starting end is located, and the capture member free end is away from the axis of the thrombus removal device and toward a distal direction.

In one embodiment, the thrombus removal device is provided with a capture segment in the axial direction of each of the connecting rods, and the capture segment includes at least two of the capture members distributed circumferentially.

In one embodiment, the capture member includes a first capture member and a second capture member; in the same capture segment, the capture member starting end of the first capture member is overlapped with the capture member starting end of the second capture member; alternatively, the capture member starting end of the first capture member and the capture member starting end of the second capture member are spaced axially along the thrombus removal device.

In one embodiment, when the capture member starting end of the first capture member and the capture member starting end of the second capture member are spaced axially along the thrombus removal device, the connecting rods connected to the first capture member and the second capture member are each in a waveform shape; the waveform shape includes wave crests and wave troughs; proximal ends of the first supporting rods of the first capture member and proximal ends of the second supporting rods of the first capture member are respectively connected to the wave crests of the different connecting rods; proximal ends of the first supporting rods of the second capture member and proximal ends of the second supporting rod of the second capture member are respectively connected to the wave troughs of the different connecting rods.

In one embodiment, the capture member is in a flat shape, an arc shape, or a waveform shape as a whole; and the first supporting rods and the second supporting rods are respectively in a rectilinear shape, a curved shape, a waveform shape or a fold-line shape.

In one embodiment, the capture member free end and a portion near the capture member free end are parallel to the axis of the thrombus removal device; alternatively, or the capture member free end and a portion near the capture member free end are away from the axis of the thrombus removal device and extend outward.

In one embodiment, the capture member has a maximum outline potion in an axial direction perpendicular to the capture member; the maximum outline potion has a larger size than that of the capture member starting end.

In one embodiment, the proximal end of the first supporting rod or the proximal end of the second supporting rod is connected with the connecting rods to form a connection point; the connection point has a width ranging from 0.05 mm to 0.5 mm.

The above thrombus removal apparatus includes a thrombus removal device and a push-pull member connected to the thrombus removal device; the thrombus removal device includes at least two connecting rods extending from the proximal end to the distal end along the thrombus removal device and capture members disposed on the connecting rods; the thrombus removal device includes a lumen structure; the connecting rods and the capture members enclose the lumen structure, where the lumen structure can effectively prevent a captured thrombus from escaping to improve the thrombus capture rate. The capture member includes a first supporting rod and a second supporting rod, and the proximal end of the first supporting rod and the proximal end of the second supporting rod are respectively connected to different connecting rods; and the end where the proximal end of the first supporting rod is located and the end where the proximal end of the second supporting rod is located form a capture member starting end, and the distal end of the first supporting rod and the distal end of the second supporting rod are connected to form a capture member free end; where, a thrombus inlet is formed between the first supporting rod and the second supporting rod of the capture member; and the capture member free end on the thrombus removal device has an opening structure which can also form a thrombus inlet. These thrombus inlets allow thrombi to enter into the lumen structure of the thrombus removal device more completely, facilitating the capture of the thrombus. Meanwhile, the capture member free end can adhere on the blood vessel wall when passing through a curved blood vessel, thus improving the success rate of thrombus removal and preventing thrombi from falling off.

### Brief Description of the Drawings

Various other advantages and benefits will become apparent to those skilled in the art upon reading the following detailed description of the embodiments. The accompanying drawings are only illustrative of the embodiments and are not to be construed as limiting. Further, the same reference numerals represent the same components throughout the accompanying drawings; where,
Fig. 1 is a structure diagram of a thrombus removal apparatus provided in a first embodiment.
Fig. 2 is another perspective diagram of the thrombus removal apparatus in Fig. 1.
Fig. 3 is a schematic diagram of a thrombus removal device in Fig. 1.
Fig. 4 is another perspective diagram of the thrombus removal device in Fig. 1.
Fig. 5 is a structure diagram of the thrombus removal apparatus in Fig. 1 after being extruded locally.
Fig. 6 is a schematic diagram showing a radial cross-section of the thrombus removal device in Fig. 1.
Fig. 7 is a schematic diagram of the thrombus removal apparatus in Fig. 1 provided with a developing unit.
Fig. 8 is another perspective diagram of the thrombus removal apparatus in Fig. 1 provided with a developing unit.
Fig. 9 is an enlarged schematic diagram of a portion B in Fig. 7.
Fig. 10 is a structure diagram of a thrombus removal apparatus provided in a second embodiment.
Fig. 11 is another perspective diagram of the thrombus removal apparatus in Fig. 10.
Fig. 12 is a schematic diagram of a thrombus removal device in Fig. 10.
Fig. 13 is another perspective diagram of the thrombus removal device in Fig. 10.
Fig. 14 is a structure diagram of a thrombus removal apparatus provided in a third embodiment.
Fig. 15 is an enlarged schematic diagram of a portion C in Fig. 14.
Fig. 16 is a schematic diagram showing a radial cross-section of the thrombus removal device in Fig. 15 at a wave trough portion.
Fig. 17 is another perspective diagram of the thrombus removal apparatus in Fig. 14.
Fig. 18 is an expanded diagram of the thrombus removal device in Fig. 14.
Fig. 19 is a schematic diagram of the thrombus removal apparatus in Fig. 14 provided with a developing unit.
Fig. 20 is a structure diagram showing that a proximal portion is connected to a push-pull member according to an embodiment.
Fig. 21 is a structure diagram of a distal portion according to an embodiment.
Fig. 22 is a structure diagram of a connection mode between a push-pull member and a thrombus removal device provided in an embodiment.
Fig. 23 is a structure diagram of a connection mode between a push-pull member and a thrombus removal device provided in another embodiment.
Fig. 24 is a structure diagram of a connection mode between a push-pull member and a thrombus removal device provided in another embodiment.
Fig. 25 is a structure diagram of a connection mode between a push-pull member and a thrombus removal device provided in a further embodiment.

### Detailed Description of the Embodiments

Exemplary embodiments will be described in more detail below with reference to the accompanying drawings. Even though the exemplary embodiments are shown in the accompanying drawings, it should be understood that the embodiments may be implemented in various forms and should not be limited. On the contrary, these embodiments are provided such that the scope can be fully conveyed to those skilled in the art.

It should be understood that the terms used herein are only illustrative of exemplary embodiments and are not intended to give any limitation. As used herein, the singular forms "alan", "one," and "the" may also include plural forms, unless otherwise specified explicitly. The terms "comprise", "include", "contain" and "have" are inclusive, and therefore indicate the existence of features, steps, operations, elements and/or components stated, but do not exclude the existence or addition of one or more other features, steps, operations, elements, components, and/or combinations thereof. The method steps, processes, and operations described herein are not to be construed as necessarily requiring them to be executed in the particular order described or illustrated, unless the order of execution is explicitly specified. It should also be understood that additional or alternative steps may be used.

Although the terms first, second, third, etc. may be used herein to describe a plurality of elements, components, regions, layers and/or sections, these elements, components, regions, layers and/or sections should not be limited by these terms. These terms may be used only to distinguish one element, component, region, layer or section from another region, layer, or section. Terms such as "first", "second", and other numerical terms when used herein do not imply a sequence or an order, unless otherwise specified explicitly. Therefore, a first element, component, region, layer, or section discussed hereafter may be termed a second element, component, region, layer, or section without departing from the teachings of the exemplary embodiments.

For the convenience of description, spatially relative terms, such as "inner", "outer", "inside", "outside", "below", "under", "over", "upside", and the like, may be used herein to describe the relation of one element or feature relative to another element (s) or feature (s) as illustrated in the drawings. Such spatially relative terms are intended to encompass different orientations of the device in use or operation in addition to the orientation depicted in the drawings. For example, if a device in the drawings is turned over, elements described as "under" or "below" other elements or features would then be oriented "over" or "upside" the other elements or features. Thus, the exemplary term "below..." may encompass both orientations of the above and below. The device may be additionally oriented (rotated 90 degrees or in other directions) and the spatially relative descriptors used herein should be interpreted accordingly.

For the convenience of description, the following description uses the terms "proximal end" and "distal end", where the "proximal end" refers to the end close to the operator and the "distal end" refers to the end away from the operator; the phrase "axial direction" should be understood to refer to the direction in which the thrombus removal apparatus is pushed in and out; the direction perpendicular to the "axial direction" is defined as a "radial direction"; the phrase "length direction" should be understood to refer to the direction in which the thrombus removal device is physically longest and, the direction perpendicular to the "length direction" is defined as a "radial direction".

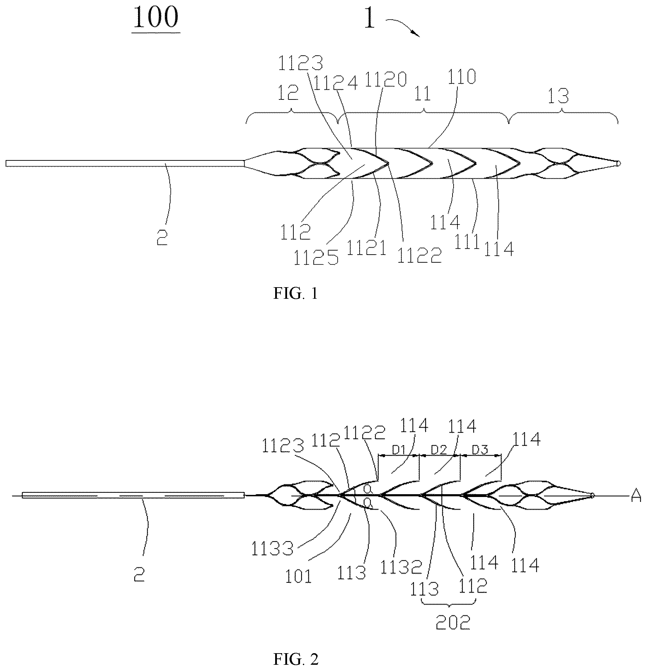

Referring to Fig. 1, the first embodiment may provide a thrombus removal apparatus 100, including a thrombus removal device 1 and a push-pull member 2; a distal end of the push-pull member 2 is connected to a proximal end of the thrombus removal device 1.

The thrombus removal device 1 includes a proximal portion 12, an intermediate portion 11 and a distal end portion 13 connected in sequence from the proximal end to the distal end; and the push-pull member 2 is connected to a proximal end of the proximal portion 12. In other embodiments, the thrombus removal device 1 may include an intermediate portion 11 only, or an intermediate portion 11 and a proximal portion 12 only, or an intermediate portion 11 and a distal portion 13 only.

The intermediate portion 11 includes at least two connecting rods 110 and 111 extending from the proximal end to the distal end along the thrombus removal device 1, and capture members 202 disposed on the connecting rods 110, 111 (see Fig. 2). The intermediate portion 11 has a lumen structure; the connecting rods 110 and 111 enclose the lumen structure with the capture members 202. At least one of the connecting rods 110 and 111 is in a rectilinear shape, a waveform shape or a fold-line shape. The connecting rods 110 and 111 may be a combination of several shapes. In this embodiment, the connecting rods 110 and 111 are both in a rectilinear shape.

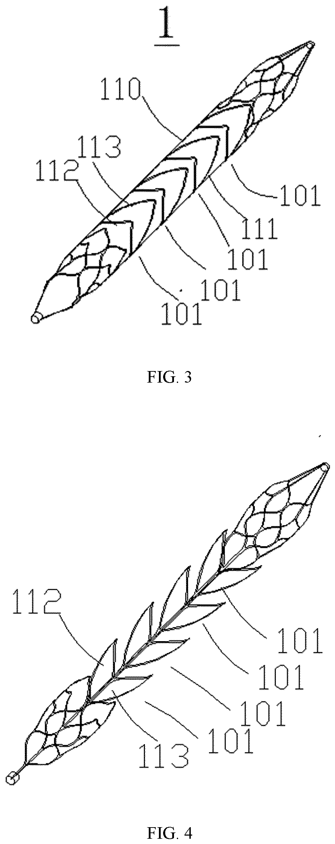

With reference to Figs. 2, 3 and 4, in this embodiment, the intermediate portion 11 is provided axially with four capture segments 101, each capture segment 101 includes capture members 202 distributed circumferentially, where each capture member 202 includes a first capture member 112 and a second capture member 113. In other embodiments, the number of the capture segments 101 may be one or more, where one capture segment 101 may include only one capture member 202 or may include three or more capture members 202 distributed circumferentially. In this embodiment, the number of connecting rods is two; and in other embodiments, the number of connecting rods may be more than two; the connecting rods are distributed along the circumferential direction of the lumen structure, and each capture member 202 may be arbitrarily connected onto at least two of the connecting rods.

Referring again to Fig. 1, the first capture member 112 includes a first supporting rod 1120 and a second supporting rod 1121, the proximal end of the first supporting rod 1120 is connected to the connecting rod 110, thus forming a first connection point 1124; the proximal end of the second supporting rod 1121 is connected to the connecting rod 111, thus forming a second connection point 1125; and the end where the proximal end of the first supporting rod 1120 is located and the end where the proximal end of the second supporting rod 1121 is located form a capture member starting end 1123; the distal end of the first supporting rod 1120 is connected to the distal end of the second supporting rod 1121 to form a capture member free end 1122. Similarly, in Fig. 2, the second capture member 113 has the same structure as the first capture member 112, and also includes a first supporting rod and a second supporting rod, and also includes a capture member starting end 1133 and a capture member free end 1132. In this embodiment, the capture member starting end 1123 of the first capture member 112 is overlapped with the capture member starting end 1133 of the second capture member 113. It is understood that the number of the supporting rods of the capture member is not limited to two, but may be three or more; and the supporting rods may be arbitrarily connected to at least two connecting rods. In this embodiment, an opening between the first supporting rod 1120 and the second supporting rod 1121 is a thrombus inlet 114.

It will be appreciated that in Figs. 1 and 2, the shape of the first supporting rod 1120 and the second supporting rod 1121 affects the overall shape of the first capture member 112, where the first supporting rod 1120 and the second supporting rod 1121 may be respectively in a rectilinear shape, a curved shape, a waveform shape or a fold-line shape; and the first capture member 112 may be in a flat shape, an arc shape, or a waveform shape as a whole. The shape of the first capture member 112 and the second capture member 113 may or may not be the same. In this embodiment, the shape of the first capture member 112 and the shape of the second capture member 113 are the same, both in an arc-shaped surface. In other embodiments, the first supporting rod 1120 or the second supporting rod 1121 may be a combination of any two of the several shapes including a rectilinear shape, a curved shape, a waveform shape or a fold-line shape.

In Fig. 2, the first capture member 112 has an included angle a with an axial cross-section A of the thrombus removal device 1 where the capture member starting end 1123 is located, and the capture member free end 1122 is away from the axis of the thrombus removal device 1 and toward a distal direction. In this embodiment, the second capture member 113 is symmetrical to the first capture member 112 with respect to the cross-section A. In particular, the second capture member 113 also has an included angle a with an axial cross-section A of the thrombus removal device 1 where the capture member starting end 1133 is located, and the capture member free end 1132 is away from the axis of the thrombus removal device 1 and toward a distal direction. It will be understood that in this embodiment, the cross-section A is an axial cross-section where the central axis of the thrombus removal device 1 is located. In other embodiments, the second capture member 113 may be not symmetrical with the first capture member 112 with respect to the cross-section A; the cross-section A may be any axial cross-section where the thrombus removal device 1 is located.

Compared with the configuration that the capture member free end 1122 is away from the axis of the thrombus removal device 1 and toward the proximal direction, the configuration way that the capture member free end 1122 is away from the axis of the thrombus removal device 1 and toward the distal direction may reduce damage to the inner wall of the blood vessel when the thrombus removal device 1 is taken back after thrombus removal. It will be understood that in other embodiments, the capture member free end 1122 may also be away from the axis of the thrombus removal device 1 and toward the proximal direction. In this embodiment, the capture member free ends of all the capture members are toward a same direction; in other embodiments, the direction of the capture member free end 1122 of the capture member may be disposed arbitrarily.

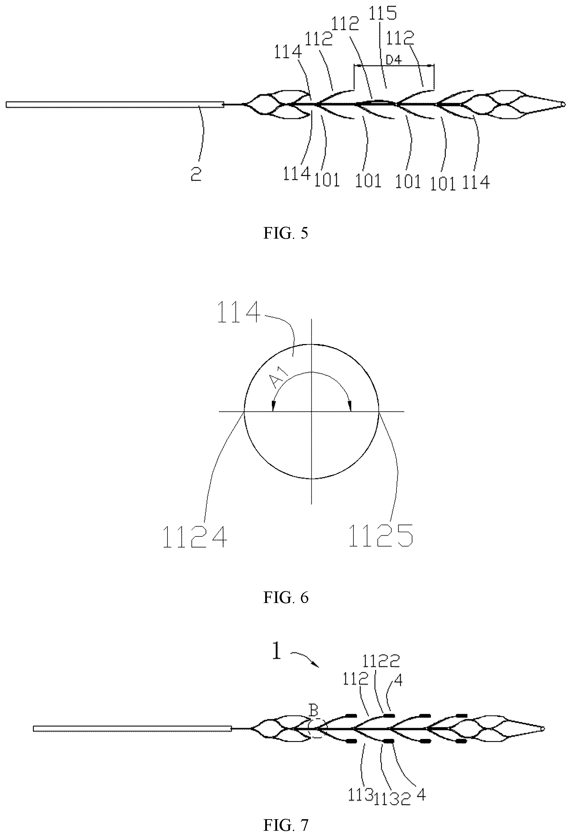

In combination with Figs. 2 and 5, there is a thrombus inlet 114 between adjacent capture segments 101. In this embodiment, each capture segment 101 includes two capture members (a first capture member 112 and a second capture member 113). Since the capture member has such a structural feature of the capture member free end, the thrombus removal device 1 has an open structure at the position of the capture member free end; such kind of open structure is a thrombus inlet 114. Thus, a capture segment 101 has two thrombus inlets 114 which are located on one side of the first capture member 112 and one side of the second capture member 113, respectively. In other embodiments, one capture segment 101 includes three or more capture members, thrombus inlets 114 are circumferentially distributed; the number of the thrombus inlets 114 is the same as the number of capture members. In this embodiment, the thrombus removal device 1 has a thrombus inlet 114 between the proximal portion 12 and the intermediate portion 11; the thrombus removal device 1 also has a thrombus inlet 114 between the distal portion 13 and the intermediate portion 11. It will be understood that if there are more thrombus inlets 114 circumferentially distributed in a capture segment 101, more thrombi can be captured accurately.

In Fig. 2, the distance between the capture member free ends 1122 on the same side of the four capture segments 101 from the proximal end to the distal end is respectively D1, D2, D3; where D1, D2, D3 are respectively the axial lengths of the corresponding capture segments 101 therein, where 2 mm <D1; D2 and D3 <12 mm. With reference to Fig. 5, when the first capture member 112 on one side in contact with a thrombus is deformed by interacting with the thrombus, a larger thrombus inlet 115 is formed between the first capture members 112 which are not affected by the thrombus at the two ends adjacent to the affected first capture member 112; and the axial length of the thrombus inlet 115 is D4, where D4 = D1 + D2; and the capture member free ends 1122 of the non-affected first capture members 112 at the two adjacent ends can be kept in an opened state and still be well adhered on the vessel wall. Therefore, even if the thrombus removal device 1 is affected by the thrombus or squeezed by the internal environment of the blood vessel, the thrombus removal device 1 may still remove the thrombus flexibly and reliably, which can improve the efficiency of thrombus removal and prevent the thrombus from escaping.

Fig. 6 is a schematic diagram showing a radial cross-section of the thrombus removal device 1 in Fig. 1. The first connection point 1124 between the first supporting rod 1120 and the connecting rod 110, and the second connection point 1125 between the second supporting rod 1121 and the connecting rod 111 are shown in Fig. 1. In Fig. 6, an arc-shaped range A1 between the first connection point 1124 and the second connection point 1125 is the size of the radial area of one thrombus inlet 114. In this embodiment, the first connection point 1124 is symmetrically arranged to the second connection point 1125 with respect to the central axis of the thrombus removal device 1; and the size of one thrombus inlet 114 accounts for half the size of the lumen structure of the thrombus removal device 1.

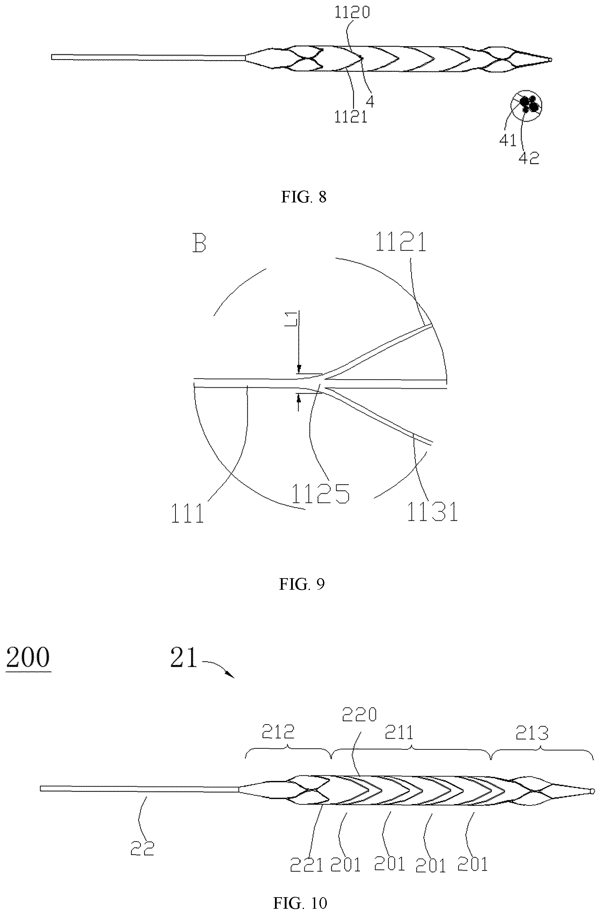

Referring to Figs. 7 and 8, the thrombus removal device 1 has a plurality of developing units 4. To clearly determine whether the capture members 112 and 113 are opened, the developing units 4 may be mounted at the capture member free ends 1122 and 1132. The material of the developing unit may be a pure metal or an alloy with higher molecular weight, such as platinum, gold, platinum-iridium alloy, and platinumtungsten alloy; and the developing unit may be sleeved on the distal end of the free end 4513. In this embodiment, the way as shown in Fig. 8 may be also used, the first supporting rod 1120 or the second supporting rod 1121 has a hole structure 41 both in the position near the distal end; and a developing material 42 is embedded into the hole structure 41 to form the developing unit 4. In this embodiment, the developing unit 4 has a quincunx shape; and in other embodiments, the developing unit 4 may be dotshaped, "8"-shaped, "0"-shaped, or "V"-shaped. Referring again to Fig. 7, in this embodiment, the capture member free ends (1122, 1132) (including the portions near the capture member free ends (1122, 1132)) are parallel to the axis of the thrombus removal device 1.

With reference to Fig. 9, the proximal end of the second supporting rod 1121 of the first capture member 112 is connected to the connecting rod 111 to form a second connection point 1125; in this embodiment, the proximal end of the second supporting rod 1131 of the second capture member 113 is also converged in the second connection point 1125; and at this time, the second connection point 1125 has a width of L1 ranging from 0.05 mm to 0.5 mm. If L1 is too large, it may make it difficult for the thrombus removal device 1 to enter into a catheter, while if L1 is too small, it may result in poor strength of the second connection point 1125. It will be understood that widths of the other connection points on the thrombus removal device 1 are also within this range.

Referring to Fig. 10, the second embodiment may provide a thrombus removal apparatus 200, including a thrombus removal device 21 and a push-pull member 22; the distal end of the push-pull member 22 is connected to the proximal end of the thrombus removal device 21.

The thrombus removal device 21 includes a proximal portion 212, an intermediate portion 211 and a distal portion 213 connected in sequence from the proximal end to the distal end; and the push-pull member 22 is connected to the proximal end of the proximal portion 212. In other embodiments, the thrombus removal device 21 may include an intermediate portion 211 only, or an intermediate portion 211 and a proximal portion 212 only, or an intermediate portion 211 and a distal portion 213 only.

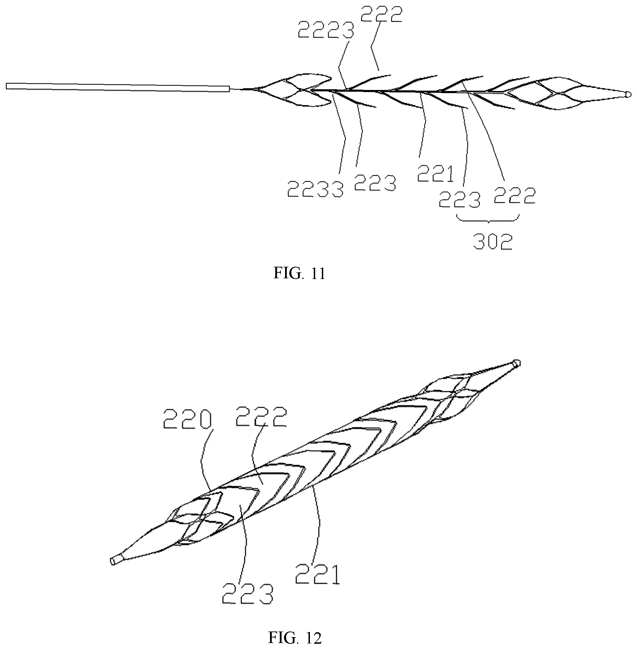

In the present embodiment, the intermediate portion 211 is axially provided with four capture segments 201. In combination with Figs. 11-13, each capture segment 201 includes a capture member 302 distributed circumferentially (see Fig. 11 in detail), the capture member 302 includes a first capture member 222 and a second capture member 223; and the capture member starting end 2223 of the first capture member 222 and the capture member starting end 2233 of the second capture member 223 are not overlapped but are axially spaced along the thrombus removal device 21. In this embodiment, the capture member starting ends of each capture member in each capture segment 201 are not overlapped, which may avoid the too wide connection points between the capture member starting ends 2223, 2233 and the connecting rods 220, 221, thus making the thrombus removal device 21 difficult to enter into the catheter.

Other structural features of this embodiment are the same as those of the thrombus removal apparatus 100 provided in the first embodiment and thus, will not be described in detail herein.

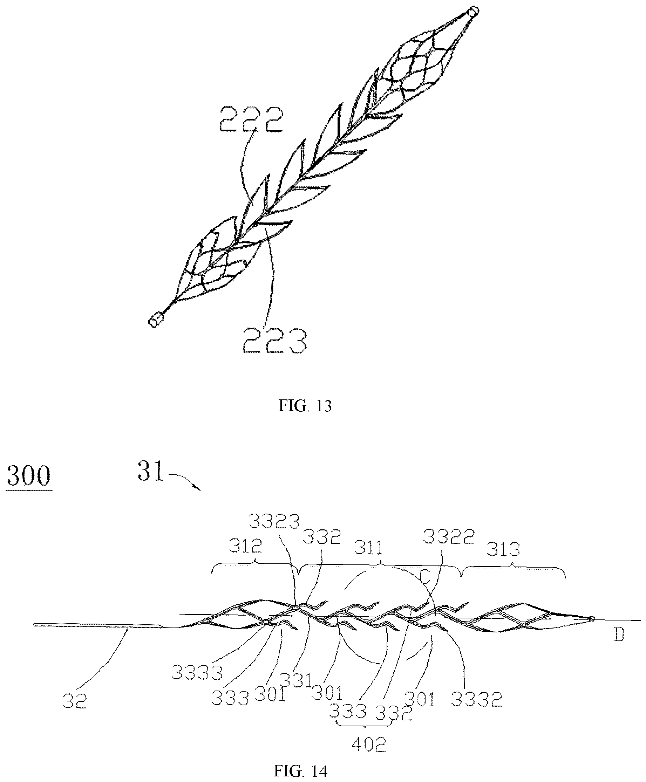

Referring to Fig. 14, the third embodiment may provide a thrombus removal apparatus 300, including a thrombus removal device 31 and a push-pull member 32; the distal end of the push-pull member 32 is connected to the proximal end of the thrombus removal device 31.

The thrombus removal device 31 includes a proximal portion 312, an intermediate portion 311 and a distal portion 313 connected in sequence from the proximal end to the distal end; and the push-pull member 32 is connected to a proximal end of the proximal portion 312. In other embodiments, the thrombus removal device 31 may include an intermediate portion 311 only, or an intermediate portion 311 and a proximal portion 312 only, or an intermediate portion 311 and a distal portion 313 only. In this embodiment, the intermediate portion 311 is provided axially with four capture segments 301, each capture segment 301 includes a capture member 402 distributed circumferentially, where the capture member 402 includes a first capture member 332 and a second capture member 333. The same as the second embodiment, the capture member starting end 3323 of the first capture member 332 and the capture member starting end 3333 of the second capture member 333 are not overlapped, but axially spaced along the thrombus removal device 31.

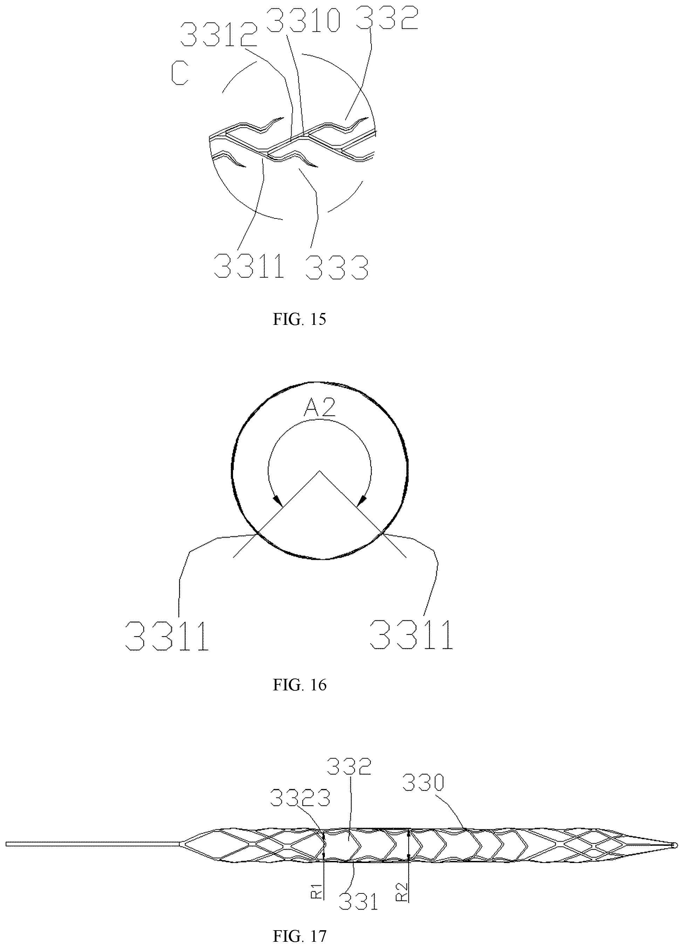

In this embodiment, the connecting rods 330 and 331 are each in a waveform shape; and the connecting rods 330 and 331 are symmetrical with each other, and the wave-shaped connecting rod 330 or 331 includes a wave crest potion and a wave trough potion. In Fig. 15, the wave-shaped connecting rod 331 includes a wave crest potion 3310 and a wave trough potion 3311; and a wave rod 3312 is connected between the wave crest potion 3310 and the wave trough potion 3311; the first capture member 332 is connected to the wave crest potion 3310, and the second capture member 333 is connected to the wave trough potion 3311.

With regard to the first capture member 332, such configuration may lengthen a thrombus capture length of the first capture member 332. At this time, the thrombus capture length of the first capture member 332 is the sum of the length of the first capture member 332 and the length of the wave rod 3312; and the thrombus capture length of the first capture member 332 is lengthened, which is equivalent to lengthening the thrombus inlet of the first capture member 332, capable of improving the thrombus removal efficiency.

Fig. 16 is a schematic diagram showing a radial cross-section of the thrombus removal device 31 in Fig. 1 at a wave trough portion 3311. When the starting end of the first capture member 332 is extended to the wave trough portions 3311 of the two connecting rods, the arc-shaped range A2 between the wave trough portions 3311 of the two connecting rods is the size of the thrombus capturing radial area of the first capture member 332. By comparison between Fig. 6 and Fig. 16, it can be seen that the arc-shaped range A2 is significantly larger than the arc-shaped range A1. Therefore, the configuration mode in this embodiment may increase the size of the thrombus capturing radial area of the first capture member 332 and may improve the thrombus removal efficiency.

In this embodiment, capture members are disposed at adjacent wave crest portions and wave trough portions in one capture segment; and in other embodiments, the first capture member 332 and the second capture member 333 may be spaced apart by one or more wave crest portions and wave trough portions along the length of the connecting rod in one capture segment; or the first capture member 332 and the second capture member 333 may be disposed at any position of the wave rod 3312. In other embodiments, the connecting rods 330, 331 may not be symmetrical with each other and may have different waveforms.

Referring again to Fig. 14, in this embodiment, the first capture member 332 and the second capture member 333 are generally in a waveform shape. Both the capture member free end 3322 of the first capture member 332 (including the portion near the first capture member free end 3322) and the capture member free end 3332 of the second capture member 333 (including the portion near the second capture member free end 3332) extent outward away from the central axis D of the thrombus removal device 31. Compared with the configuration that the capture member free end (including the portion near the capture member free end) is parallel to the axis of the thrombus removal device 31, the configuration mode in this embodiment may achieve better adherence on the vessel wall.

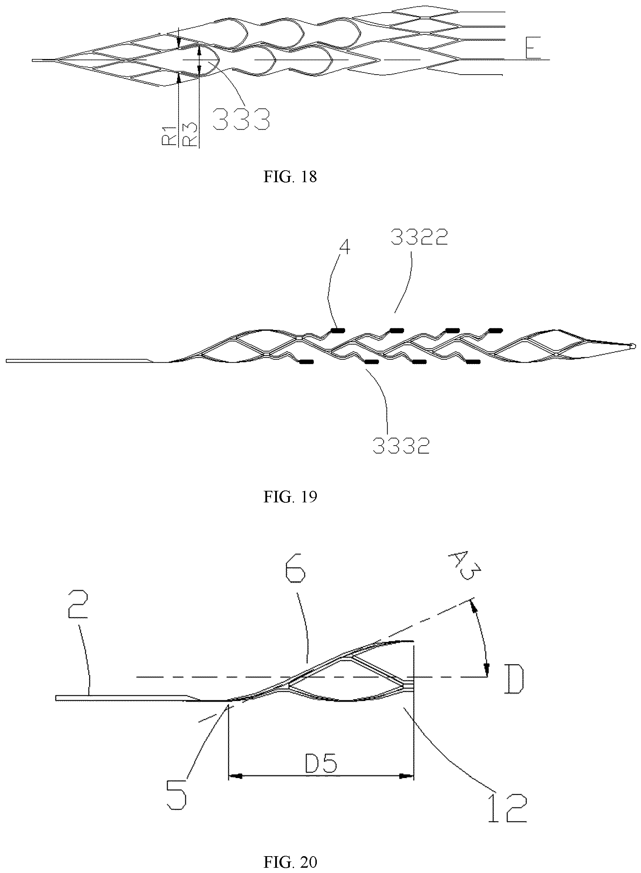

With reference to Figs. 17 and 18, in this embodiment, the structure and shape of the first capture member 332 are the same as those of the second capture member 333; the capture member starting end 3323 of the first capture member 332 (or the second capture member 333) has a length of R1; the thrombus removal device 31 has a diameter of R2, R2>R1. The second capture member 333 (or the first capture member 332) has a maximum outline potion in an axial direction E perpendicular to the capture member, and the maximum outline potion has a length R3, where R3>R1. Such configuration may increase the thrombus capturing radial width of the first capture member 332 (or the second capture member 333), thus improving the thrombus removal efficiency.

Referring to Fig. 19, the same as the first embodiment, the developing unit 4 may be mounted on the capture member free ends (3322, 3332); the capture member free ends (3322, 3332), and the portions near the capture member free ends (3322, 3332) are parallel to the axis of the thrombus removal device 31.

Other structural features of this embodiment are the same as those of the thrombus removal apparatus 100 provided in the first embodiment and thus, will not be described in detail herein.

In this embodiment, with reference to Fig. 20, when the thrombus removal device 1 further includes a proximal portion 12, the push-pull member 2 is connected to the proximal end of the proximal portion 12 to form a connection portion 5; the connection portion 5 is located on one side of the central axis D of the thrombus removal device 1. The proximal end of the proximal portion 12 has a slope 6 which forms an angle A3 with the central axis D of the thrombus removal device 1. If A3 is too large, the thrombus removal device 1 requires a large tensile force to enter into a microcatheter. If A3 is too small, the axial length D5 of the slope 6 is too long; and the potion does not participate in the thrombus removal process, *i. e.,* the effective length of the thrombus removal device 1 with the same overall length is smaller. In this embodiment, A3 is 10° to 45°.

In this embodiment, the proximal portion 12 is a self-expandable meshed stent structure, and the structure has a stronger radial supporting force than the intermediate portion 11 and the proximal portion 12. To make the radial support force of the proximal portion 12 stronger, it is possible to widen the rod width of the proximal portion 12 in the circumferential direction, or to thicken the wall thickness of the proximal portion 12 in the radial direction, or to use a densified grid structure, or to configure the radial diameter of the proximal portion 12 to be greater than that of the intermediate portion 11, or use a combination thereof. In other embodiments, the proximal portion 12 may further be a saccule-expanded mesh structure.

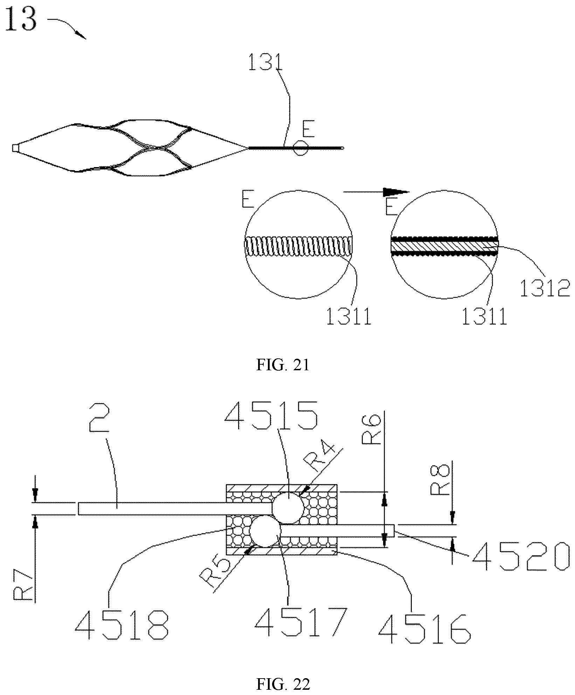

Referring to Fig. 21, when the thrombus removal device 1 further includes a distal portion 13, the distal portion 13 presents a distally-closed mesh structure for preventing thrombi from escaping. The distal end of the distal portion 13 is also provided with a damage-proof portion 131 such that the thrombus removal device 1 has better flexibility, thus facilitating the reduction of damage to blood vessels. The damage-proof portion 131 includes a flexible rod 1311 and a spring 1312 wrapped around the outer surface of the flexible rod 1311. In other embodiments, flexible rod 1311 is not included and a spring 1312 is included only. The spring 1312 is made of a metal material with a higher molecular weight, such as being wound by gold, silver, copper, and calcium. The material used has an outer diameter of 0.005-0.5 mm; the damage-proof portion 131 has an outer diameter of 0.01-0.2 mm. In other embodiments, the distal portion 13 may also be a distally-opened mesh structure.

Referring again to Fig. 1, the thrombus removal device 1 has a proximal portion 12, an intermediate portion 11 and a distal portion 13 at the same time, and the thrombus removal device 1 has a lumen structure extending therethrough from the proximal portion 12 to the distal portion 13. The thrombus removal device 1 has an overall length of 20-70 mm; the intermediate portion 11 has a length greater than the length of the proximal portion 12; and the intermediate portion 11 has a length greater than the length of the distal portion 13. The length of the intermediate portion 11 ranges from 10% to 90% of the total length of the thrombus removal device 1; and further, the length of the intermediate portion 11 ranges from 33% to 66% of the total length of the thrombus removal device 1. The thrombus removal device 1 has a maximum diameter of 2-7 mm in the axial direction.

In this embodiment, the thrombus removal device 1 may be formed by performing laser cutting on a metal tubular product (e.g., a NiTi alloy tube) with a shape memory effect and superelasticity, followed by die forming and heat treatment for shaping. Alternatively, the thrombus removal device 1 may be formed by cutting a sheet metal with a shape memory effect and superelasticity first, followed by die forming and heat treatment for shaping. The tubular product or sheet may have a thickness of 0.05-0.5 mm. Alternatively, the thrombus removal device 1 may be formed by weaving a metal wire with shape memory effect and superelasticity first, followed by die forming and heat treatment for shaping. Alternatively, the thrombus removal device 1 may also be made of a highly elastic polymer material. The above suitable materials are well known to those skilled in the art and thus, will not be described in detail herein.

The distal end of the push-pull member 2 is fixedly connected to the proximal end of the thrombus removal device 1. The connection mode between the thrombus removal device 1 and the push-pull member 2 may be welding, bonding, pressing rivet, etc, and is not limited herein. To ensure that the thrombus removal device 1 can enter into a smaller microcatheter, the diameter of the push-pull member 2 shall not exceed 0.5 mm. Furthermore, the diameter of the push-pull member 2 is 0.05-0.4 mm. The push-pull member 2 may be made of a metal with better elasticity, including stainless steel, nickeltitanium alloy, and cobalt-chromium alloy.

The connection mode between the push-pull member 2 and the thrombus removal device 1 may be referring to Figs. 22-25. In Figs. 22-25, the proximal end of the thrombus removal device 1 is connected to the distal end of the push-pull member 2 by a connecting member 4516.

In one embodiment, as shown in Fig. 22, the push-pull member 2 has a push-pull member distal member 4515; the push-pull member distal member 4515 has a maximum size R4 greater than a diameter R7 of the push-pull member 2. The thrombus removal device 1 has a thrombus removal device proximal member 4517; the thrombus removal device proximal member 4517 has a maximum size R5 greater than a diameter R8 of an adjacent connecting portion 4520 (which is a part of the proximal portion 12 of the thrombus removal device 1). The push-pull member distal member 4515 and thrombus removal device proximal member 4517 are simultaneously wrapped by a connecting member 4516 which may be a tubular product or a spring. The connecting member 4516 has a filler 4518 which will be not separated from the connecting member 4516 after being filled inside the connecting member 4516. The filler 4518 may be a glue, a molten metal, or the like. To ensure that the thrombus removal device 1 can enter into a microcatheter, the connecting member 4516 has a diameter of 0.1-0.5 mm and a length of 0.5-3 mm. In this embodiment, the push-pull member distal member 4515 and the thrombus removal device proximal member 4517 are spherical; and the sum of the maximum size R4 of the push-pull member distal member 4515 and the maximum size R5 of the thrombus removal device proximal member 4517 is greater than the inner diameter of the connecting member 4516.

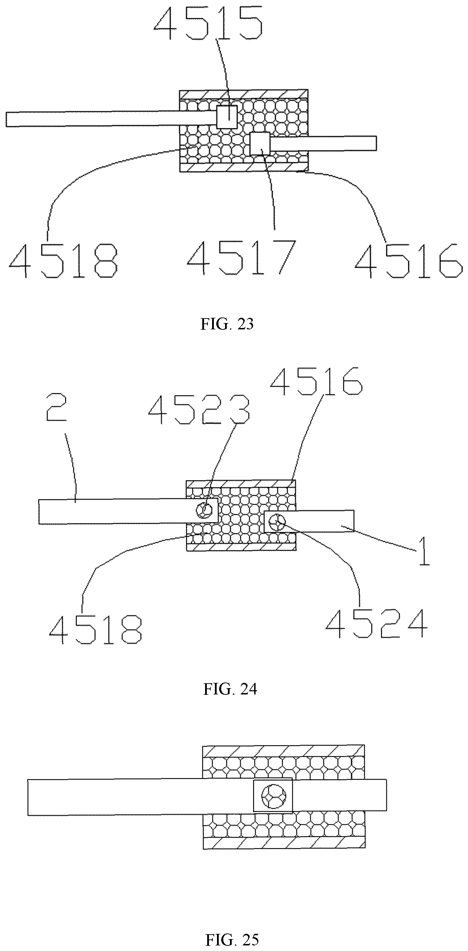

In Fig. 23, the push-pull member distal member 4515 and the thrombus removal device proximal member 4517 are squared; and the sum of the maximum size R4 of the push-pull member distal member 4515 and the maximum size R5 of the thrombus removal device proximal member 4517 is less than the inner diameter of the connecting member 4516. The push-pull member distal member 4515 and the thrombus removal device proximal member 4517 are mainly fixed by the filler 4518 in the connecting member 4516. In other embodiments, the push-pull member distal member 4515 and the thrombus removal device proximal member 4517 may be in any shape.

In another embodiment, as shown in Fig. 24, the push-pull member 2 has a hole 4523 on the distal end and the thrombus removal device 1 has a hole 4524 on the proximal end. The distal end of the push-pull member 2 and the proximal end of the thrombus removal device 1 are simultaneously wrapped by a connecting member 4516; and the connecting member 4516 may be a tubular product or a spring. The connecting member 4516 has a filler 4518 which will be not separated from the connecting member 4516 after being filled inside the connecting member 4516. The filling material may be a glue, a molten metal, or the like. In this embodiment, the hole 4523 is not overlapped with the hole 4524. In Fig. 25, the hole 4523 may be overlapped with the hole 4524.

What is described above are merely embodiments, but the protection scope is not limited thereto. Any person skilled in the art may readily envisage variations or substitutions that shall fall within the protection scope of the claims.

## Claims

1. A thrombus removal apparatus, comprising:
a thrombus removal device, further comprising:
at least two connecting rods extending from a proximal end to a distal end along the thrombus removal device,
a capture member disposed on the at least two connecting rods, and
a lumen structure, wherein the at least two connecting rods and the capture member enclose the lumen structure; and
a push-pull member connected to the thrombus removal device, wherein
the capture member comprises a first supporting rod and a second supporting rod, a proximal end of the first supporting rod and a proximal end of the second supporting rod are respectively connected to different connecting rods of the at least two connecting rods, an end where the proximal end of the first supporting rod is located and an end where the proximal end of the second supporting rod is located form a capture member starting end, and a distal end of the first supporting rod and a distal end of the second supporting rod are connected to form a capture member free end.

2. The thrombus removal apparatus of claim 1, wherein at least one of the at least two connecting rods is in a rectilinear shape, a waveform shape, or a fold-line shape.

3. The thrombus removal apparatus of claim 1, wherein the capture member has an included angle with an axial cross-section of the thrombus removal device where the capture member starting end is located, and the capture member free end is away from the axis of the thrombus removal device and toward a distal direction.

4. The thrombus removal apparatus of claim 1, wherein the thrombus removal device is provided with a capture segment in the axial direction of the at least two connecting rods and the capture segment includes at least two capture members distributed circumferentially.

5. The thrombus removal apparatus of claim 4, wherein the capture member further comprises a first capture member and a second capture member, in a same capture segment, and the capture member starting end of the first capture member is overlapped with the capture member starting end of the second capture member;
alternatively, the capture member starting end of the first capture member and the capture member starting end of the second capture member are spaced axially along the thrombus removal device.

6. The thrombus removal apparatus of claim 5, wherein, when the capture member starting end of the first capture member and the capture member starting end of the second capture member are spaced axially along the thrombus removal device, the at least two connecting rods connected to the first capture member and the second capture member are each in a waveform shape, the waveform shape comprises wave crests and wave troughs, a proximal end of the first supporting rod of the first capture member and a proximal end of the second supporting rod of the first capture member are respectively connected to the wave crests of the different connecting rods, and a proximal end of the first supporting rod of the second capture member and a proximal end of the second supporting rod of the second capture member are respectively connected to the wave troughs of the different connecting rods.

7. The thrombus removal apparatus of claim 1, wherein the capture member is in a flat shape, an arc shape, or a waveform shape as a whole; and the first supporting rod and the second supporting rod are respectively in a rectilinear shape, a curved shape, a waveform shape, or a fold-line shape.

8. The thrombus removal apparatus of claim 1, wherein the capture member free end and a portion near the capture member free end are parallel to the axis of the thrombus removal device, or
the capture member free end and a portion near the capture member free end are away from the axis of the thrombus removal device and extend outward.

9. The thrombus removal apparatus of claim 1, wherein the capture member has a maximum outline portion in a direction perpendicular to an axial direction of the capture member and the maximum outline portion has a greater size than a size of the capture member starting end.

10. The thrombus removal apparatus of claim 1, wherein the proximal end of the first supporting rod or the proximal end of the second supporting rod is connected to the at least two connecting rods to form a connection point having a width ranging from 0.05 mm to 0.5 mm.
